# EUROPÄISCHE PATENTSCHRIFT

(11) **EP 1 655 006 B1**
(45) Veröffentlichungstag und Bekanntmachung des Hinweises auf die Patenterteilung: **22.08.2007**
(21) Anmeldenummer: 05023472.3
(22) Anmeldetag: 27.10.2005
(51) Int. Cl.: A61F 13/15, G01N 3/24, G01N 19/04

(54) **Verfahren zum Prüfen der Verschlusskräfte eines auf Scherung beanspruchten Verschlusssystems**
Process for testing of peel strength of fasteners
Procédé de tester la résistance de pelage des élements de fixation

(30) Priorität: 03.11.2004 DE 102004053468
(43) Veröffentlichungstag der Anmeldung: 10.05.2006
(73) Patentinhaber: Paul Hartmann AG, 89522 Heidenheim (DE)
(72) Erfinder: Stupperich, Hans-Peter, 89522 Heidenheim (DE); Hornung, Fridmann, 73466 Lauchheim (DE); Kesselmeier, Rüdiger, Dr., 89542 Herbrechtingen (DE)
(74) Vertreter: Friz, Oliver

(56) Entgegenhaltungen:
- GB-A- 2 201 796
- US-A1- 2003 220 626
- US-A1- 2004 087 929
- US-A1- 2004 147 899

## Beschreibung

Die Erfindung betrifft ein Verfahren zum Prüfen der Verschlusskräfte eines auf Scherung beanspruchten Verschlusssystems bei absorbierenden Hygieneartikeln, wie Windeln, Gürtelwindeln, Inkontinenzwindeln und -vorlagen, durch Ausführen einer Zugprüfung und Bestimmen der hierbei auftretenden Zugkräfte, wobei das Verschlusssystem zwei flächenhafte Abschnitte umfasst, die aufeinandergelegt werden, um haftend, insbesondere klebend haftend oder mechanisch haftend, zusammenzuwirken.

Absorbierende Hygieneartikel der vorstehend genannten Art werden üblicherweise mit klebenden oder mechanisch wirkenden Verschlussmitteln ausgestattet, die ein lösbares Verschlusssystem bilden, mittels dessen der Hygieneartikel in einer bestimmungsgemäßen Position und an die individuelle Körpergestalt des Benutzers, insbesondere an seinen Hüftumfang angepasst, angelegt werden kann. Zur Prüfung des Gebrauchszustands des Hygieneartikels oder zum Wechseln des als benutzt erkannten Hygieneartikels wird das Verschlusssystem geöffnet und im ersteren Fall gegebenenfalls wieder geschlossen.

Bei den Verschlussmitteln zur Bildung der Verschlusssysteme handelt es sich in der Regel um mit Kleber beschichtete Laschen, die auf entsprechend ausgebildete Landezonen zum Schließen des Artikels klebend haftend aufgebracht werden, oder es handelt sich um mechanisch wirkende Verschlussmittel, die eine hakenbildende Verschlusskomponente und eine schlaufenbildende Verschlusskomponente aufweisen, die mechanisch haftend zusammenwirken können.

Bei der Ausbildung von Verschlusssystemen für Hygieneartikel soll einerseits eine stabile Verschlussverbindung erreicht werden, die auch Bewegungssituationen Rechnung trägt und die sich nicht ungewollt löst. Andererseits soll aber das Verschlusssystem zerstörungsfrei geöffnet werden können, wenn dies erwünscht ist, und vorzugsweise auch wiederverschließbar sein. Um die Kräfte bei Tragesituationen zu simulieren, also insbesondere in Hüftumfangsrichtung wirkende Zugkräfte, die auf die Verschlusssysteme einwirken, ist es bekannt, bei solchen Verschlusssystemen Zugprüfversuche durchzuführen, bei denen das Verschlusssystem entweder auf Abschälung ("peeling", Abzugswinkel = 180°) oder auf Scherung beansprucht wird. Es werden die Kräfte während der Ausführung einer Zugprüfung bis hin zum Lösen der Verschlussverbindung aufgezeichnet.

Der vorliegenden Erfindung liegt die Aufgabe zugrunde, die hierbei zugrundegelegten Prüfbedingungen besser an tatsächliche Tragesituationen anzupassen, um die Genauigkeit der hierbei erzielten Ergebnisse und deren Aussagegehalt zu erhöhen.

Diese Aufgabe wird bei einem Verfahren der eingangs genannten Art erfindungsgemäß dadurch gelöst, dass die zwei flächenhaften Abschnitte für die Durchführung der Zugprüfung über eine gekrümmte Fläche gelegt werden und dabei auf Zug beansprucht werden.

Die gekrümmte Fläche soll dabei einen Hüft- oder Bauchbereich eines Benutzers simulieren. Auf diese Weise werden die Prüfbedingungen weiter an eine tatsächliche Tragesituation im Gebrauch des Hygieneartikels angepasst. Es zeigt sich nämlich, dass der vom Bauch oder den Hüften eines Benutzers ausgehende Druck und die Stützung des Verschlusssystems, welche sich üblicherweise im Bauch oder Hüftbereichbereich befindet, eine Auswirkung auf die Verschlusskräfte ausübt.

Vorteilhaft ist die gekrümmte Fläche wenigstens abschnittsweise kreisbogenförmig gekrümmt, wobei der Radius des Kreisbogens veränderlich insbesondere aber konstant sein kann.

Es hat sich als zweckmäßig erwiesen, wenn die bogenförmig gekrümmte Fläche wenigstens abschnittsweise einen Krümmungsradius von 200 - 600 mm, insbesondere von 250 - 550 mm, insbesondere von 300 - 500 mm und weiter insbesondere von 350 - 450 mm aufweist.

Für die Durchführung des Zugprüfversuchs erweist es sich als vorteilhaft, wenn die flächenhaften Abschnitte direkt aus einem sie bildenden Flachmaterial herausgebildet, insbesondere herausgestanzt werden. Es wäre denkbar, dass dieses Flachmaterial als Ganzes mit Klemmen einer Zugprüfvorrichtung verbunden und auf Zug beansprucht wird. Indessen erweist es sich als vorteilhaft, wenn die flächenhaften Abschnitte mit einem biegsamen Substrat verbunden werden und zusammen mit diesem über die bogenförmig gekrümmte Fläche gelegt werden. Die flächenhaften Abschnitte werden dabei durch das biegsame Substrat vorzugsweise verlängert, so dass sie insbesondere, vergleichbar einer Gürtelschnalle, über ein Ende des biegsamen Substrats vorstehen und in diesem vorstehenden Bereich miteinander lösbar haftend zusammenwirken.

Es hat sich als zweckmäßig erwiesen, wenn die über die gekrümmte Fläche gelegte Anordnung über eine Umfangslänge von 100 mm - 400 mm, insbesondere von 150 mm - 350 mm, insbesondere von 150 mm - 300 mm in Berührungskontakt mit der gekrümmten Fläche steht.

Die vorliegende Erfindung betrifft aber auch eine Vorrichtung zur Durchführung des Verfahrens, die gekennzeichnet ist durch eine in Richtung der auszuübenden Zugkräfte erstreckte gekrümmte Fläche, gegen die das zu prüfende Verschlusssystem anlegbar ist, wenn es durch eine vorzugsweise motorisch antreibbare Einrichtung auf Zug beansprucht wird

Weitere Einzelheiten, Merkmale und Vorteile des erfindungsgemäßen Verfahrens und der erfindungsgemäßen Vorrichtung ergeben sich aus den beigefügten Patentansprüchen und der zeichnerischen Darstellung und nachfolgenden Beschreibung einer bevorzugten Ausführungsform der Erfindung. Es zeigen:
- Figur 1: eine schematische Darstellung des Prüflings;
- Figur 2: eine schematische Darstellung des Aufbaus eines Zugprüfversuchs mit einer Vorrichtung mit gekrümmter Fläche;
- Figur 3: einen Querschnitt des in Figur 1 dargestellten Prüflings entlang einer Linie A-A
- Figur 4: eine perspektivische Ansicht eines Teils einer Vorrichtung zur Durchführung einer Zugprüfung.

Nachfolgend wird eine Prüfmethode für die Ermittlung der Verschlusskräfte bei Scherbeanspruchung erläutert. Für die Durchführung der Prüfmethode kann ein Zugprüfgerät Typ Z010 / TN 2S, Messdose 100 N, erhältlich bei der Firma Zwick GmbH & Co KG, Ulm, Deutschland, mit einer Klemmbackenbreite zum Einspannen des Prüflings von 60 mm verwendet werden. Bei der Durchführung der Prüfmethode wird das zu prüfende Verschlusssystem mit einer schlaufenbildenden Komponente und einer darauf haftenden hakenbildenden Komponente über eine gekrümmte Fläche gelegt, welche eine Rundung des Bauchbereichs eines Benutzers simulieren soll. Zur Verbindung der Verschlussmittel mit den Klemmbacken des Zugprüfgeräts wird ein biegsames Substrat, beispielsweise ein einseitiges Klebeband einer bevorzugten Breite von 25 mm, das unter der Bezeichnung STA 306 bei der Firma 3M Deutschland GmbH ansässig in Neuss erhältlich ist, verwendet. Das Klebeband ist aus Polypropylen, seine Oberfläche ist beschichtet durch ein urethanmodifiziertes Siliconpolymer. Das Flächengewicht des Haftklebstoffauftrags beträgt 23 g/m². Der über die gekrümmte Fläche gelegte Prüfling, bestehend aus aufeinander haftenden flächenhaften Abschnitten der Verschlussmittel, wird durch Verwendung des Zugprüfgeräts auf Zug beansprucht, woraus sich eine scherende Beanspruchung der aneinander haftenden flächenhaften Abschnitte ergibt.

### Probenvorbereitung:

Die zu verwendenden mechanischen Verschlussmittel 1, also das schlaufenbildende Vliesstoffmaterial 2 und eine hakenbildende Komponente 3 des Verschlusssystems, werden über 24 h bei 23° C und 50 % relativer Luftfeuchte konditioniert. Es werden flächenhafte Abschnitte 6 einer Größe von 50 x 300 mm aus dem schlaufenbildenden Vliesstoffmaterial 2 ausgestanzt und sandwichartig zwischen die Enden 8 zweier einseitig klebender Klebebänder 10 einer Breite von 25 mm, die gegeneinander geklebt sind, angeordnet bzw. fixiert, so dass sich ein Überstand des Vliesstoffmaterials 2 von 50 x 250 mm ergibt. Wenn das zur Verfügung stehende Material von vornherein eine kleinere Abmessung aufweist, wird es so an dem Substrat 10 befestigt, dass sich ein Überstand ergibt, der eine 25 x 20 mm große Überdeckung mit der hakenbildenden Komponente 3 des Verschlussmittels 1 gestattet.

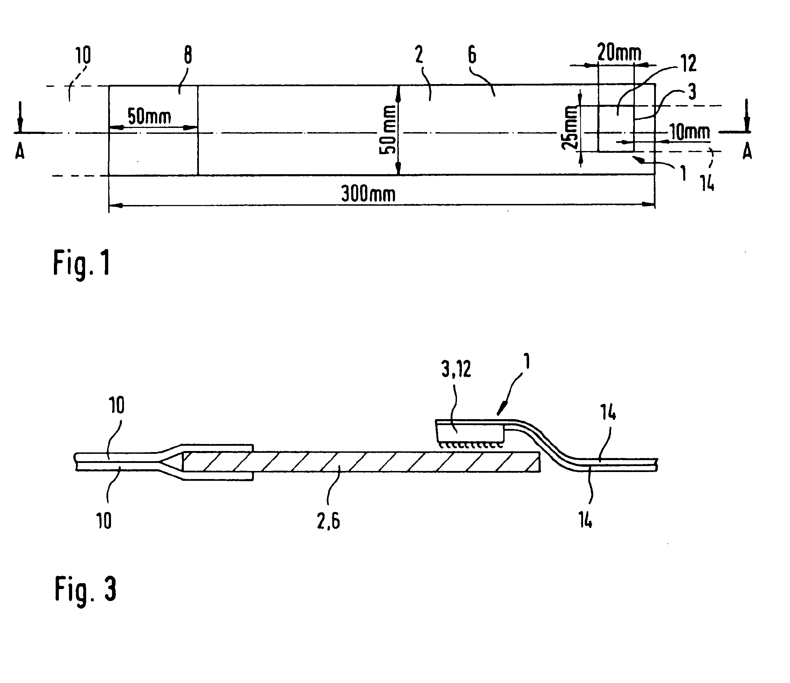

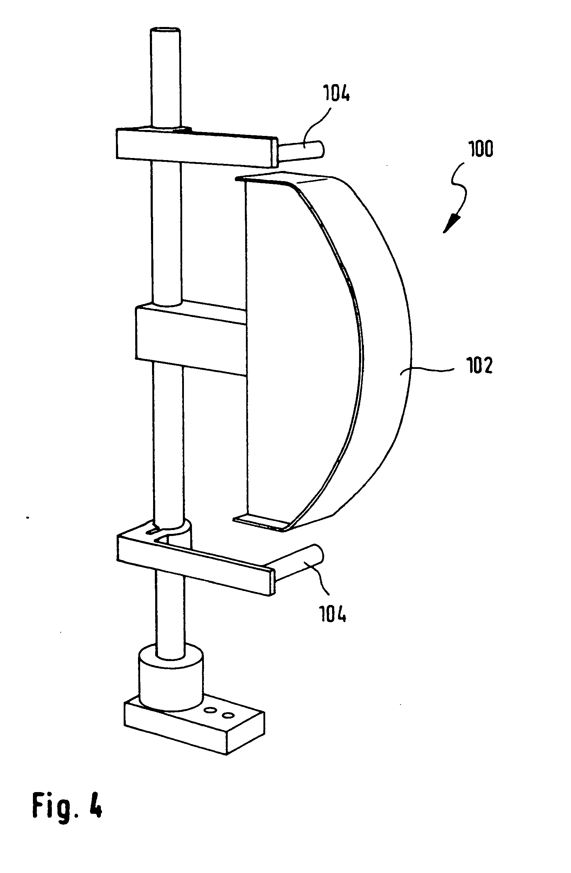

Desgleichen wird von der hakenbildenden Komponente 3 des Verschlussmittels 1 ein flächenhafter Abschnitt 12 von 25 x 20 mm ausgestanzt und wie in Figur 1 und 3 dargestellt durch zwei mit ihren Klebeflächen gegeneinander geklebte einseitige Klebebänder 14 derart fixiert, dass das oberere Klebeband die Rückseite des flächenhaften Abschnittes 12 überfängt und das untere Klebeband bündig an den flächenhaften Abschnitt 12 angrenzt.

Der flächenhafte Abschnitt 12 der hakenbildenden Komponente 3 (25 x 20 mm) wird nun auf den Abschnitt 6 des schlaufenbildenden Vliesstoffmaterials 2 aufgelegt, wobei der Abstand der hakenbildenden Komponente 3 von der Längsendkante des Vliesstoffmaterials 2 10 mm und von den seitlichen Längsrändern je 12,5 mm beträgt (s. Figur 1). Die so aufeinander gelegten flächenhaften Abschnitte 6, 12 werden durch viermaliges Anrollen mit einer 50 mm breiten und im Durchmesser 100 mm starken Rolle mit glatter Oberfläche und einem Rollengewicht von 5 kg miteinander verbunden, wobei die Anrollgeschwindigkeit 20-100 mm/sec beträgt.

### Prüfverfahren:

Das wie vorstehend beschrieben verlängerte schlaufenbildende Vliesstoffmaterial 2 wird in die untere Klemmbacke 20 des Zugprüfgeräts 22 mittig zentriert eingespannt, und das gegenüberliegende Ende der wie vorstehend beschrieben verlängerten hakenbildenden Komponente 3 wird in die bewegliche obere Klemmbacke 24 des Zugprüfgeräts 22 ebenfalls zentriert eingespannt.

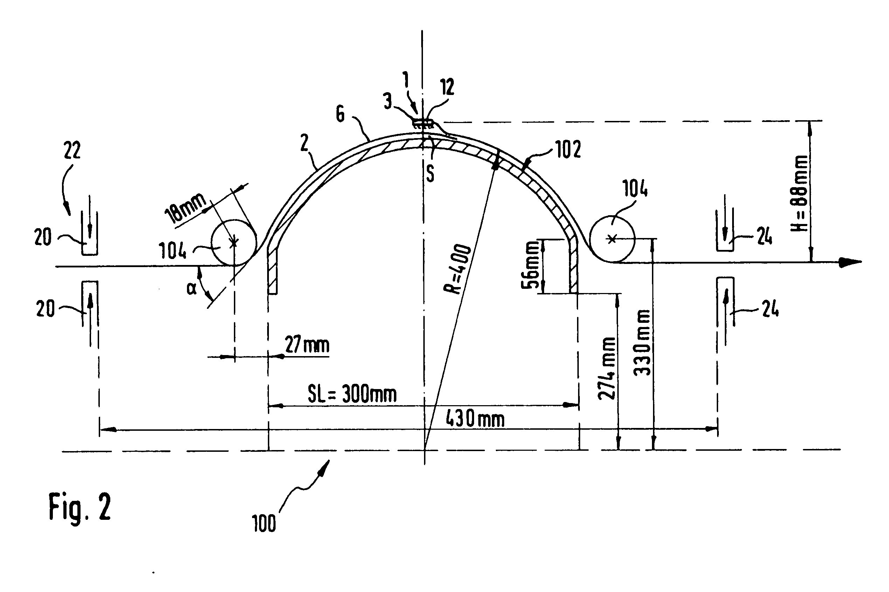

Der so eingespannte Prüfling wird über die aus Figuren 2 und 4 ersichtliche Vorrichtung 100, welche den Bauchbereich eines Benutzers simulieren soll, gelegt. Diese Vorrichtung 100 ist in Figur 4 perspektivisch dargestellt. Man erkennt eine bogenförmig gekrümmte Fläche 102 aus poliertem Stahl mit einer Rautiefe von 5 bis 25 µm und mit einem Krümmungsradius R von zumindest abschnittsweise 400 mm und einer Sehnenlänge SL von 300 mm. Des Weiteren sind oberhalb und unterhalb der gekrümmten Fläche 102 Umlenkrollen 104 vorgesehen, welche den über die gekrümmte Fläche gelegten Prüfling in die vertikale Richtung um H = 88 mm umlenken, wo er dann mit den Klemmbacken 20, 24 des nicht dargestellten Zugprüfgeräts verbunden wird. Die Umlenkung erfolgt um einen Winkel α von 60°. Hierdurch wird der Abzugswinkel im Wesentlichen tangential zu der gekrümmten Fläche 102 und konstant gehalten. Die aufeinander gelegten flächenhaften Abschnitte 6, 12 der Komponenten des Verschlussmittels 1 werden in Bezug auf die gekrümmte Fläche 102 so positioniert, dass die hakenbildende Komponente 3 mittig zentriert im Scheitelpunkt S der gekrümmten Fläche 102 zu liegen kommt.

Es wird dann die bewegliche obere Klemmbacke 24, mit der die hakenbildende Komponente verbunden ist, mit der nachfolgend angegebenen Prüfgeschwindigkeit nach oben bewegt, und es wird währenddessen die dabei auftretende Zugkraft zwischen den Klemmbacken ermittelt. Die Prüfparameter sind:

| | |
|---|---|
| - Prüfgeschwindigkeit: | 300 mm/min |
| - Einspannlänge des Prüflings: | 430 mm (s. Figur 2) |
| - Messweg: | Strecke bis zur Ablösung der Verschlussmittelkomponen ten voneinander |
| - Vorkraft: | 0,2 N |
| - Prüfanzahl: | n ≥ 6. |

Die Auswertung erfolgt dergestalt, dass die bis zum voneinander Ablösen der Verschlussmittel ermittelte Maximalkraft, gerundet auf zwei Dezimalstellen in N (Newton) notiert wird und in Form eines Mittelwerts der n-Messungen und Angabe der Standardabweichung sowie eines Minimalwerts und eines Maximalwerts angegeben wird.

Eine hakenbildende Komponente, die zusammen mit einer schlaufenbildenden Komponente eines mechanischen Verschlussmittels (beispielsweise mit einem Vliesstoff) verwendet werden kann, ist beispielsweise unter dem Handelsnamen "Microplast" 42-288-HX200-PP3 von der Firma G. Binder GmbH & Co. KG Textil- und Kunststofftechnik, Holzgerlingen, Deutschland, zu erhalten. Die Haken haben eine pilzförmige Gestalt mit ungefähr hexagonaler Fläche des Kopfs. Es befinden sich etwa 288 solcher pilzförmiger Vorsprünge pro cm². Das Material besteht aus Polypropylen und weist eine Dicke von ca. 0,42 mm auf. Es wurde durch Extrusion hergestellt. Die Höhe der pilzförmigen Erhebung gegenüber dem Grund des Materials beträgt ca. 0,26 mm. Der Abstand der Kanten der Köpfe beträgt ca. 200 µm.

## Patentansprüche

1. Verfahren zum Prüfen der Verschlusskräfte eines auf Scherung beanspruchten Verschlusssystems bei absorbierenden Hygieneartikeln, wie Windeln, Gürtelwindeln, Inkontinenzwindeln und -vorlagen, durch Ausführen einer Zugprüfung und Bestimmen der hierbei auftretenden Zugkräfte, wobei das Verschlusssystem zwei flächenhafte Abschnitte (6, 12) umfasst, die aufeinandergelegt werden, um haftend, insbesondere klebend haftend oder mechanisch haftend, zusammenzuwirken, **dadurch gekennzeichnet, dass** die zwei flächenhaften Abschnitte (6, 12) für die Durchführung der Zugprüfung über eine gekrümmte Fläche (102) gelegt werden und dabei auf Zug beansprucht werden.

2. Verfahren nach Anspruch 1, **dadurch gekennzeichnet, dass** die gekrümmte Fläche (102) wenigstens abschnittsweise kreisbogenförmig gekrümmt ist.

3. Verfahren nach Anspruch 1 oder 2, **dadurch gekennzeichnet, dass** die gekrümmte Fläche (102) wenigstens abschnittsweise einen Krümmungsradius (R) von 200 - 600 mm, insbesondere von 250 - 550 mm, insbesondere von 300 - 500 mm und weiter insbesondere von 350 - 450 mm aufweist.

4. Verfahren nach Anspruch 1, 2 oder 3, **dadurch gekennzeichnet, dass** die flächenhaften Abschnitte (6, 12) eine schlaufenbildende Komponente (2) und eine hakenbildende Komponente (3) eines mechanisch wirkenden Verschlusssystems umfassen.

5. Verfahren nach einem oder mehreren der vorstehenden Ansprüche, **dadurch gekennzeichnet, dass** die flächenhaften Abschnitte (6, 12) aus einem sie bildenden Flachmaterial herausgebildet, insbesondere ausgestanzt werden.

6. Verfahren nach einem oder mehreren der vorstehenden Ansprüche, **dadurch gekennzeichnet, dass** die flächenhaften Abschnitte (6, 12) mit einem biegsamen Substrat (14) verbunden werden und zusammen mit diesem über die bogenförmig gekrümmte Fläche (102) gelegt werden.

7. Verfahren nach Anspruch 6, **dadurch gekennzeichnet, dass** die flächenhaften Abschnitte (6, 12) durch das biegsame Substrat (14) verlängert werden.

8. Verfahren nach einem oder mehreren der vorstehenden Ansprüche, **dadurch gekennzeichnet, dass** die über die gekrümmte Fläche (102) gelegte Anordnung über eine Umfangslänge von 100 mm - 400 mm, insbesondere von 150 mm - 350 mm, insbesondere von 150 mm - 300 mm in Berührungskontakt mit der gekrümmten Fläche (102) steht.

9. Vorrichtung zur Durchführung des Verfahrens nach einem oder mehreren der vorstehenden Ansprüche, **gekennzeichnet durch** eine in Richtung der auszuübenden Zugkräfte erstreckte gekrümmte Fläche (102), gegen die das zu prüfende Verschlusssystem anlegbar ist, wenn es durch eine vorzugsweise motorisch antreibbare Einrichtung auf Zug beansprucht wird.

10. Vorrichtung nach Anspruch 9, **dadurch gekennzeichnet, dass** die gekrümmte Fläche (102) eine geschliffene oder polierte, insbesondere metallische oder metallisierte Oberfläche aufweist.

11. Vorrichtung nach Anspruch 9 oder 10, **dadurch gekennzeichnet, dass** die Rautiefe der gekrümmten Fläche (102) 5 - 25 µm beträgt.

12. Vorrichtung nach Anspruch 9, 10 oder 11, **dadurch gekennzeichnet, dass** die gekrümmte Fläche (102) wenigstens abschnittsweise kreisbogenförmig gekrümmt ist.

13. Vorrichtung nach Anspruch 12, **dadurch gekennzeichnet,** die gekrümmte Fläche wenigstens abschnittsweise einen Krümmungsradius (R) von 200 - 600 mm, insbesondere von 250 - 550 mm, insbesondere von 300 - 500 mm und weiter insbesondere von 350 - 450 mm aufweist.

14. Vorrichtung nach einem der Ansprüche 9 - 13, **dadurch gekennzeichnet**, **gekennzeichnet durch** eine Umlenkrolle (104) auf einer oder vorzugsweise beiden Seiten der gekrümmten Fläche (102), über die die zu prüfende Anordnung führbar ist.

## Claims

1. Method for testing the closure forces of a closure system subject to shear in absorbent sanitary products, such as nappies, waistband nappies, incontinence nappies and incontinence pads, by carrying out a tension test and determining the tensile forces arising in this test, the closure system comprising two two-dimensional portions (6, 12) positioned one on top of the other in order to cooperate in an adherent manner, in particular in a sticking adhesive manner or in a mechanically acting manner, **characterised in that** the two two-dimensional portions (6, 12) are positioned over a curved surface (102) for carrying out the tension test and, in so doing, are subjected to tension.

2. Method according to claim 1, **characterised in that** at least portions of the curved surface (102) are curved in the shape of a circular arc.

3. Method according to either claim 1 or claim 2, **characterised in that** at least portions of the curved surface (102) have a radius of curvature (R) of 200 to 600 mm, in particular 250 to 550 mm, in particular 300 to 500 mm and more particularly 350 to 450 mm.

4. Method according to either claim 1, 2 or 3, **characterised in that** the two-dimensional portions (6, 12) comprise a loop-forming component (2) and a hook-forming component (3) of a mechanically acting closure system.

5. Method according to one or more of the preceding claims, **characterised in that** the two-dimensional portions 6, 12 are formed, in particular stamped, from a planar material which forms them.

6. Method according to one or more of the preceding claims, **characterised in that** the two-dimensional portions (6, 12) are connected to a flexible substrate (14) and are positioned with this substrate (14) over the arc-shaped surface (102).

7. Method according to claim 6, **characterised in that** the two-dimensional portions (6, 12) are extended by the flexible substrate (14).

8. Method according to one or more of the preceding claims, **characterised in that** the arrangement positioned over the curved surface (102) is in contact with the curved surface (102) over a peripheral length of 100 mm to 400 mm, in particular 150 mm to 350 mm, in particular 150 mm to 300 mm.

9. Apparatus for carrying out the method according to one or more of the preceding claims, **characterised by** a curved surface (102) which extends in the direction of the tensile forces to be exerted and against which the closure system to be tested may be positioned when it is subjected to tension by a preferably motor-drivable device.

10. Apparatus according to claim 9, **characterised in that** the curved surface (102) has a ground or polished, in particular metallic or metal-coated upper surface.

11. Apparatus according to either claim 9 or claim 10, **characterised in that** the roughness depth of the curved surface (102) is 5 to 25 µm.

12. Apparatus according to either claim 9, 10 or 11, **characterised in that** at least portions of the curved surface (102) are curved in the shape of an arc.

13. Apparatus according to claim 12, **characterised in that** at least portions of the curved surface have a radius of curvature (R) of 200 to 600 mm, in particular 250 to 550 mm, in particular 300 to 500 mm and more particularly 350 to 450 mm.

14. Apparatus according to any one of claims 9 to 13, **characterised by** a deflection roller (104) on one or preferably both sides of the curved surface (102), over which the arrangement to be tested may be guided.

## Revendications

1. Procédé pour tester la résistance au pelage d'un système de fermeture soumis au cisaillement dans le cadre d'articles d'hygiène absorbants tels que des couches, des couches-culottes, des couches et garnitures pour incontinents, à travers la réalisation d'un essai de traction et la détermination des forces de traction qui apparaissent alors, le système de fermeture comprenant deux segments plats (6, 12) qui sont placés l'un sur l'autre afin de coopérer par adhérence, en particulier par collage ou par adhérence mécanique, **caractérisé en ce que** les deux segments plats (6, 12) sont positionnés au-dessus d'une surface courbée (102) pour la réalisation de l'essai de traction et sont ainsi soumis à la traction.

2. Procédé selon la revendication 1, **caractérisé en ce que** la surface courbée (102) est courbée au moins par endroits en forme d'arc de cercle.

3. Procédé selon la revendication 1 ou 2, **caractérisé en ce que** la surface courbée (102) présente au moins par endroits un rayon de courbure (R) compris entre 200 et 600 mm, en particulier entre 250 et 550 mm, en particulier entre 300 et 500 mm et plus particulièrement entre 350 et 450 mm.

4. Procédé selon la revendication 1, 2 ou 3, **caractérisé en ce que** les segments plats (6, 12) comprennent un composant formant des boucles (2) et un composant formant des crochets (3) d'un système de fermeture à action mécanique.

5. Procédé selon une ou plusieurs des revendications précédentes, **caractérisé en ce que** les segments plats (6, 12) sont créés à partir d'un matériau plat les formant, en particulier découpés dans celui-ci.

6. Procédé selon une ou plusieurs des revendications précédentes, **caractérisé en ce que** les segments plats (6, 12) sont reliés à un substrat souple (14) et sont positionnés en même temps que celui-ci au-dessus de la surface courbée en forme d'arc de cercle (102).

7. Procédé selon la revendication 6, **caractérisé en ce que** les segments plats (6, 12) sont prolongés par le substrat souple (14).

8. Procédé selon une ou plusieurs des revendications précédentes, **caractérisé en ce** l'agencement positionné au-dessus de la surface courbée (102) est en contact avec la surface courbée (102) sur une longueur périphérique comprise entre 100 mm et 400 mm, en particulier entre 150 mm et 350 mm, en particulier entre 150 mm et 300 mm.

9. Dispositif pour la mise en oeuvre du procédé selon une ou plusieurs des revendications précédentes, **caractérisé par** une surface courbée (102) s'étendant dans la direction des forces de traction à exercer, contre laquelle le système de fermeture à tester peut être positionné lorsqu'il est soumis à la traction par l'intermédiaire d'un dispositif de préférence à entraînement motorisé.

10. Dispositif selon la revendication 9, **caractérisé en ce que** la surface courbée (102) présente une surface meulée ou polie, en particulier métallique ou métallisée.

11. Dispositif selon la revendication 9 ou 10, **caractérisé en ce que** la profondeur de rugosité de la surface courbée (102) est comprise entre 5 et 25 µm.

12. Dispositif selon la revendication 9, 10 ou 11, **caractérisé en ce que** la surface courbée (102) est courbée au moins par endroits en forme d'arc de cercle.

13. Dispositif selon la revendication 12, **caractérisé en ce que** la surface courbée présente au moins par endroits un rayon de courbure (R) compris entre 200 et 600 mm, en particulier entre 250 et 550 mm, en particulier entre 300 et 500 mm, et plus particulièrement entre 350 et 450 mm.

14. Dispositif selon l'une des revendications 9 à 13, **caractérisé en ce qu'**il est prévu un galet de renvoi (104) sur un côté ou de préférence sur les deux côtés de la surface courbée (102), grâce auquel l'agencement à tester peut être guidé.
